# FASCICULE DE BREVET EUROPEEN

(11) **EP 2 571 430 B1**
(45) Date de publication et mention de la délivrance du brevet: **31.12.2014**
(21) Numéro de dépôt: 11726872.2
(22) Date de dépôt: 16.05.2011
(51) Int. Cl.: A61B 17/16

(54) **Porte fraise pour cotyle**
Fräserhalter für Gelenkpfanne
Reamer holder for cotyloid cavity

(30) Priorité: 20.05.2010 FR 1053908
(43) Date de publication de la demande: 27.03.2013
(73) Titulaire: Societe d'Etudes, de Recherches et de Fabrication - S.E.R.F., 69150 Decines (FR)
(72) Inventeur: DELANOUE, Elisabeth, F-69003 Lyon (FR); MONFROY, Pierre-Yves, F-69140 Rillieux La Pape (FR)
(74) Mandataire: Delorme, Nicolas
(86) Numéro de dépôt international: PCT/FR2011/051086
(87) Numéro de publication internationale: WO 2011/144851

(56) Documents cités:
- EP-A1- 0 782 840
- US-A- 4 023 572
- US-B1- 6 264 647

## Description

La présente invention se rapporte à porte-fraise chirurgical et à un ancillaire pour le fraisage d'une cavité osseuse, notamment un cotyle de hanche.

Pour l'implantation d'une prothèse, en particulier une prothèse de hanche, la cavité osseuse destinée à recevoir la prothèse doit être ajustée avec précision à la prothèse, en particulier lorsque la cavité osseuse est pathologiquement déformée. A cet effet, il est connu d'utiliser une fraise chirurgicale adaptée pour façonner l'os. Une telle fraise comporte généralement une calotte hémisphérique creuse présentant des surfaces coupantes.

Lors d'une intervention chirurgicale il est souvent nécessaire de recourir à plusieurs fraises de tailles différentes. Il est donc essentiel de disposer d'un porte-fraise permettant le changement d'une fraise facilement durant l'intervention, ceci pour éviter de devoir disposer de plusieurs outils chirurgicaux.

Pour permettre le changement d'une fraise, celle-ci présente généralement des tiges radiales disposées dans la calotte pour permettre l'accouplement en rotation de la fraise sur le porte-fraise.

Il est connu par exemple de la demande de brevet EP 0 782 840 de disposer d'un porte-fraise présentant des crans de baïonnette pour permettre d'accoupler facilement une fraise sur le porte-fraise durant l'intervention chirurgicale. Dans le porte-fraise décrit dans la demande de brevet EP 0 782 840, le blocage de la fraise dans les crans de baïonnette est réalisé à l'aide d'une pluralité d'ergots qui sont montés mobiles axialement dans des logements prévus à cet effet. Un tel porte-fraise présente plusieurs inconvénients.

Tout d'abord, ce porte-fraise présente une complexité due à l'ajustement et l'assemblage de plusieurs éléments mobiles. Cette complexité est synonyme de coûts élevés de production et peut difficilement être miniaturisé en vue d'une utilisation de ce porte-fraise en chirurgie mini-invasive.

De plus, il peut arriver lors d'une intervention chirurgicale que des débris d'os ou de tissus viennent bloquer le mouvement d'un ergot dans son logement, ceci ayant pour effet de rendre difficile l'accouplement de la fraise avec le porte-fraise ou le changement de celle-ci. Le blocage d'un ergot rallonge ainsi la durée de l'intervention, au préjudice du patient.

Par ailleurs, les instruments chirurgicaux et notamment les portes-fraises sont réutilisables et doivent pour cela être nettoyés et stérilisés. La présence d'éléments mobiles dans un porte-fraise complique notablement le nettoyage et la stérilisation : par exemple, le logement de l'ergot mobile est peu ou pas accessible lors du nettoyage et de la stérilisation. Ainsi, la réutilisation d'un porte-fraise peut donc constituer une source de contamination du patient.

La présente invention a pour objet de remédier aux différents inconvénients cités précédemment. Dans ce contexte technique, la présente invention a pour objet de fournir un porte-fraise facilement nettoyable et stérilisable, en particulier qui ne nécessite pas le démontage d'un élément mobile. La présente invention a également pour objet de fournir un porte-fraise ayant une structure simple à fabriquer et à utiliser.

A cet effet, la présente invention se rapporte à un porte-fraise, destiné à coopérer avec une fraise chirurgicale pour le façonnage d'une cavité osseuse, pourvu d'une tête porte-fraise comportant une couronne dans laquelle sont ménagées au moins deux encoches traversantes et formant des crans de baïonnette adaptées pour recevoir chacune une tige radiale de la fraise chirurgicale, chaque encoche comportant successivement une portion d'insertion conçue pour l'introduction de la tige radiale et une portion de retenue conçue pour immobiliser la tige radiale selon un axe longitudinal de la couronne, le porte-fraise comportant en outre des moyens de blocage de la tige radiale dans la portion de retenue, le porte-fraise étant caractérisé en ce que les moyens de blocage comportent une paroi élastiquement déformable conçue pour occuper une position déformée adaptée pour permettre le passage de la tige radiale de la portion d'insertion vers la portion de retenue, et une position de repos adaptée pour bloquer par encliquetage la tige radiale dans la portion de retenue.

Ainsi, un porte-fraise selon l'invention est simple à mettre en oeuvre lors d'une intervention chirurgicale : la paroi élastiquement déformable permet un blocage ou un déblocage rapide de la fraise chirurgicale dans chaque encoche du porte-outil. Le déblocage et/ou le changement d'une fraise chirurgicale accouplée au porte-fraise s'effectuent simplement par déformation de la paroi élastiquement déformable et, durant l'intervention, des débris d'os ou de tissus ne risquent pas de perturber une telle opération.

Le nettoyage et la stérilisation du porte-fraise est simple et efficace puisque la paroi élastiquement déformable des moyens de blocage permet de supprimer l'ergot de verrouillage et son logement habituellement utilisés et qui sont sources de contaminations potentielles.

Enfin, le porte-fraise, grâce à sa conception simple, peut avoir des dimensions réduites pour répondre aux besoins de la chirurgie mini-invasive.

Dans un mode de réalisation de l'invention, la paroi élastiquement déformable comporte un bossage faisant saillie dans la portion de retenue et formant butée de blocage de la tige radiale. Un bossage est simple à réaliser et permet d'obtenir des moyens de blocage dont toutes les parties sont accessibles lors du nettoyage ou de la stérilisation.

Selon une caractéristique de l'invention, la paroi élastiquement déformable s'étend le long de la portion de retenue.

Selon une possibilité, la paroi élastiquement déformable est réalisée sous la forme d'une lame élastique disposée dans la portion de retenue.

Dans un mode de réalisation de l'invention, la lame élastique fait partie intégrante de la couronne et est séparée de celle-ci par une fente.

Dans une forme de réalisation, la lame élastique présente une extrémité libre comportant le bossage.

Selon une possibilité, chaque portion d'insertion s'étend selon une direction sensiblement parallèle à un axe longitudinal de la couronne et en ce que chaque portion de retenue s'étend selon une direction sensiblement perpendiculaire à un axe longitudinal de la couronne.

Dans un mode de réalisation, un logement adapté pour recevoir la tige radiale est ménagé dans le fond de la portion de retenue.

Selon une possibilité, le porte-fraise comporte quatre encoches.

Enfin, la présente invention concerne également un ancillaire pour le fraisage d'une cavité osseuse comportant un porte-fraise selon l'invention et comportant en outre une fraise chirurgicale présentant une calotte agencée pour fraiser une surface osseuse, notamment un cotyle de hanche, et au moins une tige radiale, rapportée sur la calotte, destinée à être introduite dans la portion d'insertion d'une encoche du porte-fraise et à être bloquée dans la portion de retenue de l'encoche par un mouvement relatif de rotation de la fraise chirurgicale par rapport à la tête porte-fraise.

L'invention sera bien comprise à l'aide de la description détaillée qui suit en référence au dessin schématique annexé, représentant à titre d'exemple non limitatif une forme d'exécution d'un ancillaire et d'un porte-fraise, dans lequel :
La figure 1 est une vue en perspective d'un ancillaire pour le fraisage ;
la figure 2 est une vue en perspective d'un porte-fraise ;
la figure 3 est une vue de côté d'une tête porte-fraise ;
la figure 4 est une autre vue en perspective de l'ancillaire ;

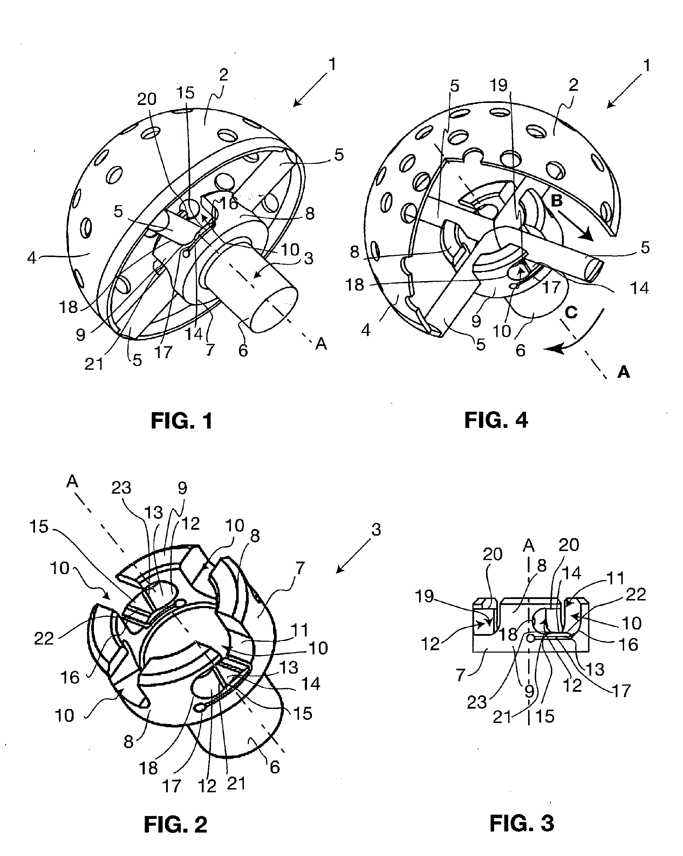

L'ancillaire 1, illustré à la figure 1, comporte une fraise chirurgicale 2 accouplée avec un porte-fraise 3. La fraise chirurgicale 2 comporte une calotte 4 hémisphérique creuse adaptée pour façonner une surface osseuse, en particulier un cotyle de hanche. La fraise chirurgicale 2 comporte quatre tiges radiales 5 cylindriques rapportées sur la calotte 4 et disposées en croix dans la partie creuse de celle-ci. Ces tiges radiales 5 permettent d'accoupler la fraise chirurgicale 2 avec le porte-fraise 3.

Le porte-fraise 3, illustré à la figure 2, comporte un arbre 6, partiellement représenté sur les figures 1, 2 et 3, permettant la transmission d'un mouvement de rotation à l'ancillaire 1 et sur lequel est montée une tête 7 porte-fraise, représentée sur la figure 3. La tête 7 porte-fraise comporte une couronne 8 présentant une paroi cylindrique 9 dans laquelle sont ménagées quatre encoches 10. Les encoches 10 traversent la paroi cylindrique 9 et sont disposées à 90° les unes des autres. Les encoches 10 forment des crans de baïonnette, sensiblement en forme de L, adaptés pour recevoir chacune une tige radiale 5 de la fraise chirurgicale 2, comme illustré à la figure 1. Chaque encoche 10 comporte successivement une portion d'insertion 11 conçue pour l'introduction de la tige radiale 5 et une portion de retenue 12 conçue pour immobiliser la tige radiale 5 selon un axe longitudinal A de la couronne 8. Chaque portion d'insertion 11 s'étend selon une direction sensiblement parallèle à l'axe longitudinal A et chaque portion de retenue 12 s'étend selon une direction sensiblement perpendiculaire à l'axe longitudinal A.

Le porte-fraise 3 comporte en outre des moyens de blocage de la tige radiale 5 dans la portion de retenue 12. Ces moyens de blocage comportent une paroi élastiquement déformable 13 conçue pour occuper, d'une part, une position déformée permettant le passage de la tige radiale 5 de la portion d'insertion 11 vers la portion de retenue 12, et d'autre part une position de repos adaptée pour bloquer par encliquetage la tige radiale 5 dans la portion de retenue 12. La paroi élastiquement déformable 13 comporte un bossage 14 faisant saillie dans la portion de retenue 12 et formant une butée de blocage de la tige radiale 5. La paroi élastiquement déformable 13 est réalisée sous la forme d'une lame élastique 15 s'étendant selon une direction sensiblement perpendiculaire à l'axe longitudinal A. La lame élastique 15 présente une extrémité libre 16 comportant le bossage 14 et vient de matière avec la couronne 8. Afin de réaliser la lame élastique 15, une fente 17, illustrée sur les figures, sensiblement parallèle à la portion de retenue 12 est ménagée dans la couronne 8 et débouche dans l'encoche 10. Ainsi, la lame élastique 15 vient de matière avec la couronne 8 au niveau du fond 18 de la portion de retenue 12, s'étend le long de la portion de retenue 12 et présente son extrémité libre 16 située au niveau de la jonction entre la portion de retenue 12 et la portion d'insertion 11. La portion de retenue 12 présente un espace d'accueil 19 destiné à recevoir la tige de retenue 5 et délimité par le fond 18, la lame élastique 15 et une surface interne 20 de la portion de retenue 12 située en regard de la lame élastique 15.

En l'absence de tige radiale 5 dans l'encoche 10, la lame élastique 15 occupe la position de repos représentée sur les figures 2 et 3.

Sur le porte-fraise 3 représenté sur les figures, la lame élastique 15 est située du côté de l'arbre 6 mais il est bien entendu que les positions de la lame élastique 15 et de la surface interne 20 pourraient être inversées entres-elles : il suffit pour cela de choisir convenablement la position de la fente 17.

Afin de recevoir la tige radiale 5 lorsque la fraise chirurgicale 2 est accouplée avec le porte-fraise 3, un logement 21 est ménagé dans le fond 18 de la portion de retenue 12.

Chaque encoche 10 présente des dimensions adaptées à la tige radiale 5 de la fraise chirurgicale 2 choisie pour être accouplée au porte-fraise 3. En particulier, la largeur de l'espace d'accueil 19 est adapté au diamètre de la tige radiale 5 et la longueur de la portion de retenue 12 est au moins égale au diamètre de la tige radiale 5.

Enfin, dans un mode de réalisation non représenté sur les figures, les moyens de blocage peuvent comporter en outre une protubérance ou un évidement disposé dans la portion de retenue 12 en regard de la paroi élastiquement déformable 13.

La figure 4 illustre les étapes successives d'accouplement d'une fraise chirurgicale 2 avec un porte-fraise 3. Sur la figure 4, la calotte 4 de la fraise chirurgicale 2 est partiellement coupée pour faciliter la compréhension du dessin.

Afin d'assurer l'accouplement de la fraise chirurgicale 2 avec le porte-fraise 3, la fraise chirurgicale 2 est positionnée de façon à permettre à chaque tige radiale 5 d'être translatée dans une portion d'insertion 11 d'une encoche 10 selon la direction représentée par la flèche B de la figure 4 sensiblement parallèle à l'axe longitudinal A, pour amener celles-ci en contact avec les extrémités libres 16 des lames élastiques 15 correspondantes. La calotte 4 de la fraise chirurgicale 2 est ensuite soumise à un mouvement de rotation relatif par rapport au porte-fraise 3, indiqué par la flèche C, de manière à ce que les tiges radiales 5 coopèrent avec chaque bossage 14 pour induire une déformation de chaque lame élastique 15. Le bossage 14 présente avantageusement une pente 22 orientée de manière à minimiser le couple de force nécessaire pour obtenir la déformation de la lame élastique 15. Lors de cette déformation, chaque lame élastique 15 s'écarte de la surface interne 20 correspondante et chaque tige radiale 5 pénètre dans chaque espace d'accueil 19. Le mouvement de rotation de la calotte 4 est maintenu jusqu'à ce que chaque tige radiale 5 vienne se positionner dans le logement 21 correspondant, dans la position représentée par la figure 1. Les lames élastiques 15 peuvent alors reprendre leur position de repos. Dans cette position, les tiges radiales 5 sont en appui sur la lame élastique 15, le fond 18 et la surface interne 20 de manière à ce que, lorsque le porte-fraise 3 est mis en rotation dans le sens inverse de celui indiqué par la flèche C, la fraise chirurgicale 2 est également mise en rotation dans le même sens. Dans la position représentée sur la figure 1, chaque tige de radiale 5 est bloquée en rotation, dans le sens inverse de celui indiqué par la flèche C, par le bossage 14.

Pour séparer la fraise chirurgicale 2 et le porte-fraise 3 il suffit d'immobiliser le porte-fraise 3 et d'effectuer une rotation de la fraise chirurgicale 2 dans le sens inverse de celui indiqué par la flèche C, en fournissant un couple de force suffisant pour obtenir la déformation de chaque lame élastique 15. Pour réduire ce couple de force le bossage 14 comporte une pente 23 orientée convenablement.

Ainsi, le porte-fraise 3 selon l'invention est simple à réaliser et ses dimensions peuvent être réduite pour permettre d'effectuer des opérations de chirurgie mini-invasive. Le nettoyage et la stérilisation d'un porte-fraise 3 est simple puisque toutes les surfaces susceptibles d'être souillées sont facilement accessibles sans nécessiter de démontage. En particulier la fente 17 peut être plus facilement nettoyée et stérilisée que les logements des ergots d'un porte-fraise classique.

Bien entendu, les exemples de réalisations évoqués ci-dessus ne présentent aucun caractère limitatif et d'autres détails et améliorations peuvent être apportés au porte-fraise 3 ou à l'ancillaire 1 selon l'invention, sans pour autant sortir du cadre de l'invention .C'est ainsi notamment que le nombre d'encoches pourrait être différent de quatre, ou que la lame élastique associée à chaque encoche pourrait ne pas faire partie intégrante de la couronne du porte-fraise.

## Revendications

1. Porte-fraise (3), destiné à coopérer avec une fraise chirurgicale (2) pour le façonnage d'une cavité osseuse, pourvu d'une tête (7) porte-fraise comportant une couronne (8) dans laquelle sont ménagées au moins deux encoches (10) traversantes et formant des crans de baïonnette adaptées pour recevoir chacune une tige radiale (5) de la fraise chirurgicale (2), chaque encoche (10) comportant successivement une portion d'insertion (11) conçue pour l'introduction de la tige radiale (5) et une portion de retenue (12) conçue pour immobiliser la tige radiale (5) selon un axe longitudinal (A) de la couronne (8), le porte-fraise (3) comportant en outre des moyens de blocage de la tige radiale (5) dans la portion de retenue (12), le porte-fraise (3) étant **caractérisé en ce que** les moyens de blocage comportent une paroi élastiquement déformable (13) conçue pour occuper une position déformée adaptée pour permettre le passage de la tige radiale (5) de la portion d'insertion (11) vers la portion de retenue (12), et une position de repos adaptée pour bloquer par encliquetage la tige radiale (5) dans la portion de retenue (12).

2. Porte-fraise (3) selon la revendication 1, **caractérisé en ce que** la paroi élastiquement déformable (13) comporte un bossage (14) faisant saillie dans la portion de retenue (12) et formant butée de blocage de la tige radiale (5).

3. Porte-fraise (3) selon la revendication 1 ou 2, **caractérisé en ce que** la paroi élastiquement déformable (13) s'étend le long de la portion de retenue (12).

4. Porte-fraise (3) selon l'une quelconque des revendications 1 à 3, **caractérisé en ce que** la paroi élastiquement déformable (13) est réalisée sous la forme d'une lame élastique (15) disposée dans la portion de retenue (12).

5. Porte-fraise (3) selon les revendications 2 et 4, **caractérisé en ce que** la lame élastique (15) présente une extrémité libre (16) comportant le bossage (14).

6. Porte-fraise (3) selon la revendication 4 ou 5, **caractérisé en ce que** la lame élastique (15) fait partie intégrante de la couronne (8) et est séparée de celle-ci par une fente (17).

7. Porte-fraise (3) selon l'une quelconque des revendications 1 à 6, **caractérisé en ce qu'**un logement (21) adapté pour recevoir la tige radiale (5) est ménagé dans le fond (18) de la portion de retenue (12).

8. Porte-fraise (3) selon l'une quelconque des revendications 1 à 7, **caractérisé en ce que** les moyens de blocage comportent en outre une protubérance ou un évidement disposé dans la portion de retenue (12) en regard de la paroi élastiquement déformable (13).

9. Porte-fraise (3) selon l'une quelconque des revendications 1 à 8, **caractérisé en ce que** chaque portion d'insertion (11) s'étend selon une direction sensiblement parallèle à un axe longitudinal (A) de la couronne (8) et **en ce que** chaque portion de retenue (12) s'étend selon une direction sensiblement perpendiculaire à un axe longitudinal (A) de la couronne (8).

10. Ancillaire (1) pour le fraisage d'une cavité osseuse comportant un porte-fraise (3) selon l'une des revendications 1 à 9 et comportant en outre une fraise chirurgicale (2) présentant une calotte (4) agencée pour fraiser une surface osseuse, notamment un cotyle de hanche, et au moins une tige radiale (5) rapportée sur la calotte (4) destinée à être introduite dans la portion d'insertion (11) d'une encoche (10) du porte-fraise (3) et à être bloquée dans la portion de retenue (12) de l'encoche (10) par un mouvement relatif de rotation de la fraise chirurgicale (2) par rapport à la tête porte-fraise (3).

## Patentansprüche

1. Fräsenhalter (3), der ausgelegt ist, um mit einer chirurgischen Fräse (2) zusammenzuarbeiten, um einen Knochenhohlraum zu bilden, ausgestattet mit einem Fräsenhalter-Kopf (7), der eine Krone (8) umfasst, in der mindestens zwei Querschlitze (10) angeordnet sind und Bajonettkerben bilden, die ausgelegt sind, um jeweils einen radialen Stift (5) der chirurgischen Fräse (2) aufzunehmen, wobei jeder Schlitz (10) aufeinander folgend einen Einführungsabschnitt (11) umfasst, der für die Einführung des radialen Stifts (5) entworfen ist, und einen Rückhalteabschnitt (12), der entworfen ist, um den radialen Stift (5) gemäß einer Längsachse (A) der Krone (8) zu immobilisieren, wobei der Fräsenhalter (3) außerdem Mittel zur Blockierung des radialen Stifts (5) im Rückhalteabschnitt (12) umfasst, wobei der Fräsenhalter (3) **dadurch gekennzeichnet ist, dass** die Blockierungsmittel eine elastisch verformbare Wand (13) umfassen, die entworfen ist, um eine verformte Position einzunehmen, die ausgelegt ist, um den Durchgang des radialen Stifts (5) vom Einführungsabschnitt (11) in den Rückhalteabschnitt (12) zu ermöglichen, und einer Ruheposition, die ausgelegt ist, um durch Schnappverbindung den radialen Stift (5) im Rückhalteabschnitt (12) zu blockieren.

2. Fräsenhalter (3) nach Anspruch 1, **dadurch gekennzeichnet, dass** die elastisch verformbare Wand (13) eine Nabe (14) umfasst, die im Rückhalteabschnitt (12) hervorsteht und einen Anschlag zur Blockierung des radialen Stifts (5) bildet.

3. Fräsenhalter (3) nach Anspruch 1 oder 2, **dadurch gekennzeichnet, dass** sich die elastisch verformbare Wand (13) entlang dem Rückhalteabschnitt (12) erstreckt.

4. Fräsenhalter (3) nach einem der Ansprüche 1 bis 3, **dadurch gekennzeichnet, dass** die elastisch verformbare Wand (13) in Form eines elastischen Blatts (15) durchgeführt ist, das im Rückhalteabschnitt (12) angeordnet ist.

5. Fräsenhalter (3) nach Anspruch 2 und 4, **dadurch gekennzeichnet, dass** das elastische Blatt (15) ein freies Ende (16) aufweist, das die Nabe (14) umfasst.

6. Fräsenhalter (3) nach Anspruch 4 oder 5, **dadurch gekennzeichnet, dass** das elastische Blatt (15) ein integraler Bestandteil der Krone (8) ist, und von dieser durch einen Schlitz (17) getrennt ist.

7. Fräsenhalter (3) nach einem der Ansprüche 1 bis 6, **dadurch gekennzeichnet, dass** eine Aufnahme (21), die ausgelegt ist, um den radialen Stift (5) aufzunehmen, auf dem Boden (18) des Rückhalteabschnitts (12) angeordnet ist.

8. Fräsenhalter (3) nach einem der Ansprüche 1 bis 7, **dadurch gekennzeichnet, dass** die Blockierungsmittel außerdem einen Vorsprung oder eine Aussparung umfassen, die im Rückhalteabschnitt (12), gegenüber der elastisch verformbaren Wand (13), angeordnet ist.

9. Fräsenhalter (3) nach einem der Ansprüche 1 bis 8, **dadurch gekennzeichnet, dass** sich jeder Einführungsabschnitt (11) gemäß einer im Wesentlichen parallelen Richtung zu einer Längsachse (A) der Krone (8) erstreckt, und dadurch, dass sich jeder Rückhalteabschnitt (12) gemäß einer im Wesentlichen senkrechten Richtung zu einer Längsachse (A) der Krone (8) erstreckt.

10. Hilfsvorrichtung (1) für das Fräsen eines Knochenhohlraums, umfassend einen Fräsenhalter (3) nach einem der Ansprüche 1 bis 9 und außerdem umfassend eine chirurgische Fräse (2), die eine Kappe (4) aufweist, die angeordnet ist, um eine Knochenfläche, insbesondere eine Hüftgelenkpfanne, zu fräsen, und mindestens einen radialen Stift (5), der auf die Kappe (4) gesetzt ist, der ausgelegt ist, um in den Einführungsbereich (11) eines Schlitzes (10) des Fräsenhalters (3) eingeführt zu werden, und um im Rückhaltebereich (12) des Schlitzes (10) durch eine relative Drehbewegung der chirurgischen Fräse (2) mit Bezug auf den Fräsenhalter-Kopf (3) blockiert zu werden.

## Claims

1. A reamer handle (3), intended to cooperate with a surgical reamer (2) for shaping a bone cavity, provided with a reamer handle head (7) comprising a crown (8) in which are preserved at least two through notches (10) and forming bayonet holes each suitable for receiving a radial stem (5) of the surgical reamer (2), each notch (10) successively comprising an inserting portion (11) designed for introducing the radial stem (5) and a retaining portion (12) designed for immobilizing the radial stem (5) according to a longitudinal axis (A) of the crown (8), the reamer handle (3) further comprising means for blocking the radial stem (5) in the retaining portion (12), the reamer handle (3) being **characterized in that** the blocking means comprise an elastically deformable wall (13) designed to occupy a deformed position suitable for allowing the passage of the radial stem (5) of the inserting portion (11) towards the retaining portion (12), and a rest position suitable for blocking the radial stem (5) by snap-fitting into the retaining portion (12).

2. The reamer handle (3) according to claim 1, **characterized in that** the elastically deformable wall (13) comprises a boss (14) protruding in the retaining portion (12) and forming a stop for blocking the radial stem (5).

3. The reamer handle (3) according to claim 1 or 2, **characterized in that** the elastically deformable wall (13) extends along the retaining portion (12).

4. The reamer handle (3) according to any one of claims 1 to 3, **characterized in that** the elastically deformable wall (13) is achieved in the form of an elastic blade (15) disposed in the retaining portion (12).

5. The reamer handle (3) according to claims 2 and 4, **characterized in that** the elastic blade (15) exhibits a free end (16) comprising the boss (14).

6. The reamer handle (3) according to claim 4 or 5, **characterized in that** the elastic blade (15) is an integral part of the crown (8) and is separated from the latter by a slit (17).

7. The reamer handle (3) according to any one of claims 1 to 6, **characterized in that** a housing (21) suitable for receiving the radial stem (5) is preserved in the bottom (18) of the retaining portion (12).

8. The reamer handle (3) according to any one of claims 1 to 7, **characterized in that** the blocking means further comprise a protuberance or a recess disposed in the retaining portion (12) facing the elastically deformable wall (13).

9. The reamer handle (3) according to any one of claims 1 to 8, **characterized in that** each inserting portion (11) extends along a direction which is substantially parallel with a longitudinal axis (A) of the crown (8) and **in that** each retaining portion (12) extends along a direction which is substantially perpendicular to a longitudinal axis (A) of the crown (8).

10. An ancillary (1) for reaming a bone cavity comprising a reamer handle (3) according to any of claims 1 to 9 and further comprising a surgical reamer (2) exhibiting a dome (4) arranged to ream a bone surface, in particular a hip acetabulum, and at least one radial stem (5) added on the dome (4) intended to be introduced in the inserting portion (11) of a notch (10) of the reamer handle (3) and to be blocked in the retaining portion (12) of the notch (10) by a relative rotation movement of the surgical reamer (2) with respect to the reamer handle (3) head.
